# EUROPÄISCHE PATENTANMELDUNG

(11) **EP 3 501 572 A1**
(43) Veröffentlichungstag der Anmeldung: **26.06.2019**
(21) Anmeldenummer: 17209695.0
(22) Anmeldetag: 21.12.2017
(51) Int. Cl.: A61M 5/148, A61M 5/155

(54) **FLUIDLIEFERVORRICHTUNG**

(71) Anmelder: de Gaudenzi, Luigi, 78234 Engen (DE)
(72) Erfinder: de Gaudenzi, Luigi, 78234 Engen (DE)
(74) Vertreter: Uexküll & Stolberg

(57) **Zusammenfassung**

Eine Fluidliefervorrichtung (1), insbesondere für Medikamente in flüssiger Form, weist einen flexiblen Innenbehälter (2), der zur Aufnahme eines Fluids (20) eingerichtet ist und mit einem Anschluss (12) in Verbindung steht, und einen Außenbehälter (4) auf. Der Außenbehälter (4) umgibt den Innenbehälter (2) zumindest teilweise, wobei zwischen dem Außenbehälter (4) und dem Innenbehälter (2) ein gasdichter Zwischenraum (8) angeordnet ist. Aus einem Flüssiggasreservoir (6) kann mittels einer von außen betätigbaren Öffnungseinrichtung (19) über einen Auslass (18) Flüssiggas (22) in den Zwischenraum (8) eingelassen werden, so dass sich in dem Zwischenraum (8) der Dampfdruck des Flüssiggases ausbildet. Dadurch kann das Fluid (20) mit einer weitgehend konstanten Förderrate zu dem Anschluss (12) geliefert werden.

## Beschreibung

Die Erfindung betrifft eine Fluidliefervorrichtung, insbesondere für Medikamente in flüssiger Form, die z.B. in einem Infusionssystem benutzt werden kann.

Es gibt implantierbare Infusionspumpen mit zwei Kammern, die durch eine flexible Membran voneinander getrennt sind. Eine der Kammern enthält ein Flüssiggas, d.h. ein Flüssigkeit/Dampf-Gemisch, das im Gleichgewicht steht und einen Druck (nämlich den Dampfdruck des Flüssiggases) auf die Membran ausübt. Der Dampfdruck ist unabhängig vom aktuellen Volumen dieser Kammer. In der anderen Kammer befindet sich z.B. eine Infusionslösung, die je nach Bauart über einen Port an der Haut des Patienten nachgefüllt werden kann. Derartige Infusionspumpen sind z.B. in DE 195 09 634 C1 offenbart und ermöglichen eine weitgehend konstante Förderrate der Infusionslösung zu dem Patienten.

Aus US 4,048,994 ist ein extern nutzbares Infusionssystem bekannt, das ein Fluid aus einem flexiblen Innenbehälter über einen Anschluss in dessen unterem Bereich ausgeben kann. Der Innenbehälter ist von einem flexiblen Außenbehälter umgeben. Der Zwischenraum zwischen dem Innenbehälter und dem Außenbehälter kann mit Druckgas befüllt werden, um auf das Fluid in dem Innenbehälter einen Druck auszuüben. Das Druckgas wird über einen Einlass (mit Rückschlagventil) in den Zwischenraum eingeführt und über einen Auslass (ebenfalls mit Rückschlagventil) daraus abgelassen. Die Handhabung ist wegen der Gasanschlüsse relativ umständlich.

Es ist Aufgabe der Erfindung, ein generell flexibel und einfach zu handhabendes Infusionssystem zu schaffen, das auf unkomplizierte Weise ein Fluid mittels eines weitgehend konstanten Drucks liefern kann und das sich vor Gebrauch gut lagern lässt.

Diese Aufgabe wird gelöst durch die Fluidliefervorrichtung mit den Merkmalen des Anspruchs 1. Vorteilhafte Ausgestaltungen der Erfindung ergeben sich aus den Unteransprüchen.

Die erfindungsgemäße Fluidliefervorrichtung ist z.B. für Medikamente in flüssiger Form geeignet und weist einen flexiblen Innenbehälter auf, der zur Aufnahme eines Fluids eingerichtet ist und mit einem Anschluss in Verbindung steht. Der Begriff "Fluid" ist hier sehr allgemein zu verstehen und erstreckt sich z.B. auf Infusionslösungen, aber auch allgemeiner auf Flüssigkeiten, thixotrope Flüssigkeiten, fließfähige Pulver, Emulsionen, Suspensionen, Gase oder Mischungen davon. Ein Außenbehälter umgibt den Innenbehälter zumindest teilweise. Zwischen dem Außenbehälter und dem Innenbehälter ist ein gasdichter Zwischenraum angeordnet. Der Anschluss für den Innenbehälter ist von außen her zugänglich; er kann sich z.B. in der Nähe des Außenbehälters befinden oder auch über eine Leitung (z.B. einen längeren Schlauch) mit dem Innenbehälter verbunden sein. Ferner ist ein Flüssiggasreservoir vorgesehen, das einen in den Zwischenraum führenden Auslass aufweist. Der Auslass lässt sich mit Hilfe einer von außen (d.h. von außerhalb des Außenbehälters) betätigbaren Öffnungseinrichtung öffnen.

Das Flüssiggasreservoir kann bereits im Auslieferungszustand der Fluidliefervorrichtung mit einem Flüssiggas befüllt sein. Eine spätere Befüllung ist ebenfalls denkbar.

Im Auslieferungszustand der Fluidliefervorrichtung kann der Innenbehälter mit einem Fluid vorbefüllt sein. Eine Befüllung mit dem Fluid durch den Benutzer ist ebenfalls möglich, z.B. mit einer pharmazeutischen Zusammensetzung wie einer Infusionslösung unmittelbar vor Anwendung der Fluidliefervorrichtung.

Unter einem Flüssiggas ist hier eine Substanz zu verstehen, die in einem abgeschlossenen Volumen sowohl als Flüssigkeit als auch als Gas vorliegt. Dabei stellt sich als Druck der Dampfdruck gemäß der gegebenen Temperatur ein. In der Praxis wählt man Substanzen aus, bei denen bei Zimmertemperatur der Dampfdruck etwas (bis wenige bar) über Atmosphärendruck liegt, z.B. ausgewählte Hydrofluoralkane, Propan, n-Butan, Isobutan oder auch Mischungen solcher Substanzen. Wenn man das Volumen vergrößert, verdampft mehr Flüssigkeit (solange genug Substanzmenge zur Verfügung steht), aber der Druck bleibt konstant.

Das Flüssiggasreservoir bildet zunächst, d.h. solange die Öffnungseinrichtung für den Auslass geschlossen ist, ein geschlossenes Volumen, in dem als Druck der Dampfdruck des Flüssiggases herrscht, aber nicht auf den Innenbehälter wirkt.

Wenn der Benutzer die Öffnungseinrichtung öffnet, tritt Flüssiggas über den Auslass in den gasdichten Zwischenraum über, so dass sich in dem Zwischenraum ebenfalls der Dampfdruck des Flüssiggases einstellt. Dies kann einige Zeit dauern, da flüssiges Flüssiggas Verdampfungswärme aufnehmen muss, um in den gasförmigen Zustand überzugehen. Wenn ein Gleichgewicht erreicht ist, drückt der Dampfdruck des Flüssiggases auf den flexiblen Innenbehälter, so dass auf ein in dem Innenbehälter befindliches Fluid ein praktisch konstanter Druck einwirkt, eben dieser Dampfdruck, der auch unabhängig von der aktuellen Füllmenge des Innenbehälters ist. Der Dampfdruck kann über die Zusammensetzung des Flüssiggases vorbestimmt werden und hängt dann im Wesentlichen nur von der Temperatur ab. Da auf das Fluid ein praktisch konstanter Druck wirkt, ist es möglich, das Fluid aus dem Innenbehälter mit praktisch konstanter Förderrate (konstantem Durchfluss) zu dem Anschluss zu liefern.

Beim Verdampfen des Flüssiggases sinkt zwar die Temperatur, aber dieser Effekt ist nach einer kurzen Anlaufzeit (z.B. einige Minuten), während der sich der Zwischenraum erstmalig mit Flüssiggas füllt, sehr gering. Denn bei üblichen Förderraten für das Fluid ist nur noch eine geringe Verdampfungsrate des Flüssiggases erforderlich, und die dafür notwendige Wärmezufuhr wird über die Wärmekapazität des im Innenbehälter vorhandenen Fluids und auch durch Wärmetransport von außen bereitgestellt.

Die Öffnungseinrichtung kann z.B. als Kunststoffteil mit einer Sollbruchstelle ausgestaltet sein, das bei Einwirkung einer tangential wirkenden Kraft bricht und den Auslass freigibt.

Der flexible Innenbehälter ist vorzugsweise aus einem Kunststoffmaterial gefertigt, das für das Flüssiggas impermeabel und (zumindest für die Gebrauchsdauer) inert ist und das für Anwendungen mit dem verwendeten Fluid verträglich bzw. zugelassen ist. Beispiele für geeignete Materialien sind Polyamid (PA), Polyaryletherketone (PAEK) und Tetrafluorethylen/Hexafluorpropylen-Copolymer (FED). Bei Bedarf kommen auch Beschichtungen sowohl auf der Seite des Zwischenraums wie auch auf der Seite des Fluids in Betracht, z.B. zellkompatible Beschichtungen oder Beschichtungen mit Nanomaterialien.

Die erfindungsgemäße Fluidliefervorrichtung kann eine Durchfluss-Einstelleinrichtung aufweisen, die zum Einstellen des Durchflusses des von der Fluidliefervorrichtung über den Anschluss gelieferten Fluids eingerichtet ist. Am Anschluss herrscht Luftdruck (oder liegt z.B. der Blutdruck eines Patienten vor), im Innenbehälter der Dampfdruck des Flüssiggases, so dass längs der Verbindung zwischen dem Innenbehälter und dem Anschluss eine weitgehend konstante Druckdifferenz besteht. Daher lässt sich bereits mit einer einfachen Einstelleinrichtung oder einem Durchflussbegrenzer, womit der Strömungswiderstand verändert werden kann, ein weitgehend konstanter Durchfluss erreichen.

Wenn die Fluidliefervorrichtung eine Durchfluss-Regeleinrichtung aufweist, lassen sich auch Schwankungen im Dampfdruck kompensieren. Eine vorteilhafte Durchfluss-Regeleinrichtung steht mit dem Zwischenraum in Verbindung (so dass der Druck des Gases im Zwischenraum direkt darauf einwirken kann) und ist dazu eingerichtet, bei steigendem Druck des Gases im Zwischenraum eine Durchlassöffnung in der Verbindung zu dem Anschluss zu verkleinern und entsprechend bei sinkendem Druck die Durchlassöffnung zu vergrößern, was eine Regelung bewirkt und die Fluidliefervorrichtung für weite Bereiche temperaturunabhägig macht.

Vorteilhaft ist ferner eine Sicherheitseinrichtung, die dazu eingerichtet ist, einen Übertritt von Flüssiggas zu dem Anschluss zu verhindern. Dies kann eine Einrichtung sein, die bei Überschreiten eines vorgegebenen Grenzdruckes in dem Innenbehälter die Verbindung zu dem Anschluss blockiert. Einfacher, jedoch erst nach Entleerung des Innenbehälters wirksam, ist ein flexibler Übergang vom Innenbehälter zu einer zu dem Anschluss führenden Infusionsleitung, der sich noch innerhalb des dem Druck des Flüssiggases ausgesetzten Zwischenraums befindet und von diesem Druck nach Entleerung des Innenbehälters flachgedrückt wird, so dass über etwaige Perforationen in der Wandung des Innenbehälters kein Flüssiggas in die Infusionsleitung gelangen kann.

Die Verbindung zu dem Anschluss kann ferner einen Filter enthalten, z.B. einen für die Verwendung mit Infusionsleitungen üblichen Filter.

Der Innenbehälter kann bereits im Auslieferungszustand der Fluidliefervorrichtung mit einem Fluid befüllt sein, z.B. einem Fluid, das eine pharmazeutische Zusammensetzung aufweist. Dazu zählen Infusionslösungen. In diesem Fall nimmt der Innenbehälter ein der Menge des Fluids entsprechendes Volumen ein, wodurch auch das Volumen des Zwischenraums bestimmt ist, sofern der Außenbehälter eine feste Form hat.

Wenn der Außenbehälter eine feste Form hat, kann sein Querschnitt z.B. rund, rechteckig, polygonal oder elliptisch sein, aber auch andere Formen aufweisen. Je höher der Dampfdruck des Flüssiggases bei Zimmertemperatur ist, umso vorteilhafter ist eine kreisförmige Querschnittsform.

Bei einer festen Form des Außenbehälters ist ein anfänglich geringes Volumen des Zwischenraums vorteilhaft. Denn vor dem Öffnen der Öffnungseinrichtung für den Auslass des Flüssiggasreservoirs herrscht im Zwischenraum vorzugsweise ein Druck, der etwa so groß ist wie der Luftdruck. Das lässt sich am einfachsten erreichen, wenn der Zwischenraum Luft enthält. Wenn nun das Flüssiggas in den Zwischenraum gelassen wird, stellt sich ein Gleichgewicht ein, das auch durch das Fremdgas "Luft" (im Wesentlichen Sauerstoff und Stickstoff) beeinflusst wird und daher die Konstanz des auf den Innenbehälter einwirkenden Drucks beeinträchtigt. Je kleiner das anfängliche Volumen des Zwischenraumes ist, umso geringer ist der Luftanteil und umso geringer ist die darauf zurückzuführende Druckabweichung.

Wenn der Zwischenraum im Auslieferungszustand der Fluidliefervorrichtung anstatt mit Luft mit Gas entsprechend dem Flüssiggas gefüllt ist (vorzugsweise mit Gas derselben Substanz wie das Flüssiggas), um einen Gegendruck gegen den äußeren Luftdruck bereitzustellen, tritt das oben genannte Problem nicht auf. Dieses Gas kann z.B. bei der Fertigung der Fluidliefervorrichtung, wenn auch der Innenbehälter befüllt wird, in den Zwischenraum mit einem Druck von 1,0 bar eingefüllt werden. Dafür benötigte Öffnungen an dem Außenbehälter werden anschließend verschlossen.

Bei anderen Ausführungsformen ist der Außenbehälter nicht starr, sondern flexibel. Dazu kann der Außenbehälter unter Verwendung eines Kunststofffolienmaterials gefertigt sein, z.B. aus Polyamid (PA) oder Polyethylenterephthalat (PET). Von Vorteil ist dabei, wenn der Außenbehälter vor Betätigung der Öffnungseinrichtung für den Auslass des Flüssiggasreservoirs zumindest teilweise an dem Innenbehälter anliegt, d.h. wenn die Form des Außenbehälters der Form des Innenbehälters angeglichen ist. In diesem Fall ist das anfängliche Volumen des Zwischenraums gering, und der oben erläuterte Einfluss auf den Dampfdruck des Flüssiggases ist sehr klein, selbst wenn sich in dem Zwischenraum Luft befindet. Sofern der Innenbehälter nicht überfüllt ist, entspricht unter diesen Voraussetzungen der Druck im Innenbehälter und der Druck im Zwischenraum vor Betätigung der Öffnungseinrichtung für den Auslass des Flüssiggasreservoirs dem Luftdruck und nach Betätigung der Öffnungseinrichtung (wenn sich der Zwischenraum prall mit Flüssiggas füllt) dem Dampfdruck des Flüssiggases. Dies gilt unabhängig von der Füllmenge des Innenbehälters.

Daher eignen sich Ausführungsformen mit flexiblem Außenbehälter besonders gut, wenn der Benutzer den Innenbehälter selbst mit Fluid befüllt, was über den Anschluss erfolgen kann (vorzugsweise vor dem Einlassen des Flüssiggases in den Zwischenraum). Der Benutzer braucht sich dabei nicht um das Gas oder den Druck im Zwischenraum zu kümmern und kann eine beliebige Menge an Fluid in den Innenbehälter einfüllen. Diese Menge ist nur durch das Maximalvolumen des flexiblen Innenbehälters begrenzt.

Wie schon erwähnt, weisen der Innenbehälter und/oder der Außenbehälter vorzugsweise ein Kunststoffmaterial auf. Besonders vorteilhaft ist es, wenn sowohl der Innenbehälter als auch der Außenbehälter transparent sind, denn dann kann man auf einen Blick die Menge an Fluid in dem Innenbehälter erfassen. Geeignete Materialien sind beispielsweise für den Innenbehälter Polyamid (PA), Polyaryletherketone (PAEK) und Tetrafluorethylen/Hexafluorpropylen-Copolymer (FED), für einen starren Außenbehälter Polymethylmethacrylat (PMMA), MMA-Copolymere wie AMMA (mit Acrylonitril), Polymethylacrylatmethylimid (PMMI) oder Polycarbonat (PC) und für einen flexiblen Außenbehälter Polyamid (PA), Polyaryletherketone (PAEK) und Polyethylenterephthalat (PET). Diese Materialien sind alle transparent.

In einer vorteilhaften Ausführungsform ist die erfindungsgemäße Fluidliefervorrichtung als Infusionssystem eingerichtet, das extern an einem Patienten verwendet wird. Dabei steht der flexible Innenbehälter über eine Infusionsleitung mit dem Anschluss in Verbindung. Der Anschluss ist vorzugsweise als Teil einer Luer-Lock-Verbindung gestaltet, so dass er sich leicht an eine Infusionsnadel ankoppeln lässt. Die Infusionsleitung kann mit einem Durchflussbegrenzer und/oder einem Partikelfilter versehen sein. Ein solches Infusionssystem ist vorzugsweise als Einwegartikel konzipiert.

Wenn das verwendete Fluid eine pharmazeutische Zusammensetzung aufweist, kann es beispielsweise die aus der folgenden Liste ausgewählten Substanzen enthalten, auch in Kombination: Analgetika, Antibiotika, Antipsychotika, Blutkomponenten, Chemotherapeutika zur Behandlung von Krebserkrankungen, monoklonale Antikörper, Sedativa, Supportiva für die parenterale Ernährung, Supportiva für die enterale Ernährung, pharmazeutische Zubereitungen zur Behandlung von Stoffwechselerkrankungen oder Autoimmunerkrankungen, pharmazeutische Zubereitungen für die Durchführung von Zelltherapien.

Die erfindungsgemäße Fluidliefervorrichtung lässt sich preisgünstig herstellen, ist bei der Anwendung sehr flexibel und kann je nach Ausführungsform auch vom Benutzer mit einer weitgehend beliebigen Menge an Fluid befüllt werden. Solange sich das Flüssiggas nur in dem Flüssiggasreservoir befindet, herrscht in dem Zwischenraum kein Überdruck (oder zumindest kein nennenswerter Überdruck), so dass sich die Fluidliefervorrichtung gut aufbewahren lässt. Bei der Anwendung wird das Flüssiggas in den Zwischenraum gelassen. Dann baut sich im Zwischenraum ein weitgehend konstanter Druck auf, nämlich der Dampfdruck des Flüssiggases, mit dem das Fluid ohne großen technischen Aufwand mit weitgehend konstanter Rate geliefert wird, z.B. zu einer Infusionsnadel bei einem Patienten.

Die große Vielzahl möglicher Formen und Materialien für die Komponenten der Fluidliefervorrichtung und bei der Auswahl des Flüssiggases (worüber sich der Druckbereich vorwählen lässt) erlauben eine Anwendung der Erfindung auf zahlreichen technischen Gebieten, nicht nur im medizinischen Bereich.

Im Folgenden wird die Erfindung anhand von Ausführungsbeispielen weiter erläutert. Die Figuren zeigen in
- Figur 1: eine schematische Ansicht einer Ausführungsform der erfindungsgemäßen Fluidliefervorrichtung nach Befüllung eines Innenbehälters mit einem Fluid,
- Figur 2: die Fluidliefervorrichtung aus Figur 1 nach dem Einlass von Flüssiggas in einen Zwischenraum,
- Figur 3: eine Ansicht ähnlich wie in Figur 2, wobei auch der obere Bereich des Innenbehälters mit einer Sicherheitseinrichtung gezeigt ist,
- Figur 4: eine schematische Ansicht einer weiteren Ausführungsform der erfindungsgemäßen Fluidliefervorrichtung, ähnlich wie Figur 1, und
- Figur 5: schematische Ansichten einer Öffnungseinrichtung für den Auslass des Flüssiggasreservoirs, und zwar in Teil (a) vor dem Öffnen, in Teil (b) nach dem Öffnen und in Teil (c) in vergrößerter Ansicht vor (links) und kurz nach (rechts) dem Öffnen.

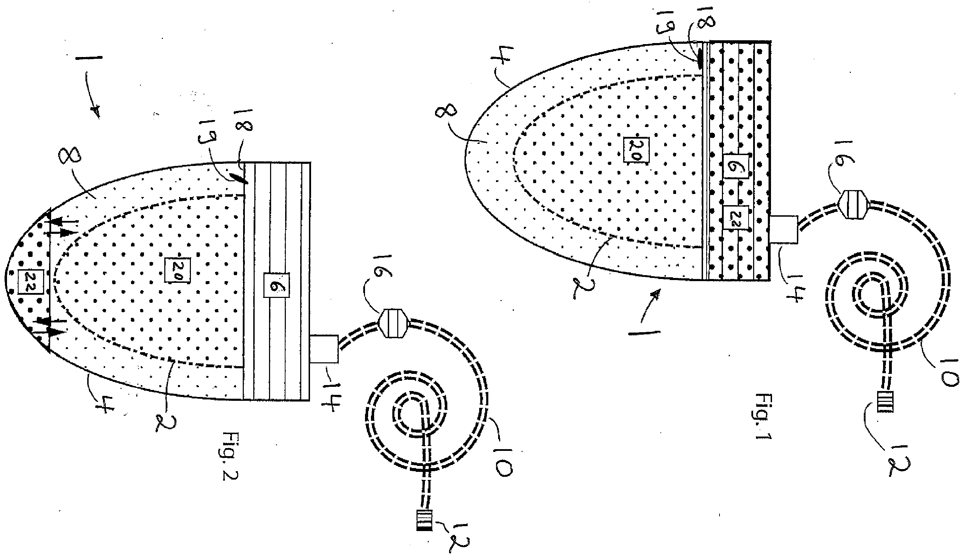

In Figur 1 ist in einer schematischen Längsschnittansicht eine Ausführungsform einer Fluidliefervorrichtung dargestellt, die mit 1 bezeichnet ist. Im Ausführungsbeispiel ist die Fluidliefervorrichtung 1 als Infusionssystem ausgestaltet, das es ermöglicht, einem Patienten eine pharmazeutische Zusammensetzung in Fluidform mit weitgehend konstanter Rate zuzuführen.

Die Fluidliefervorrichtung 1 weist einen flexiblen Innenbehälter 2 und einen Außenbehälter 4 auf, der im Ausführungsbeispiel eine starre Form hat. Im oberen Teil des Außenbehälters 4 befindet sich ein Flüssiggasreservoir 6. Zwischen dem Innenbehälter 2 und dem Außenbehälter 4 ist ein Zwischenraum 8 angeordnet. Das Flüssiggasreservoir 6 kann auch auf den oberen Bereich des Außenbehälters 4 aufgesetzt sein. Der Zwischenraum 8 ist ebenso wie das Flüssiggasreservoir 6 gasdicht.

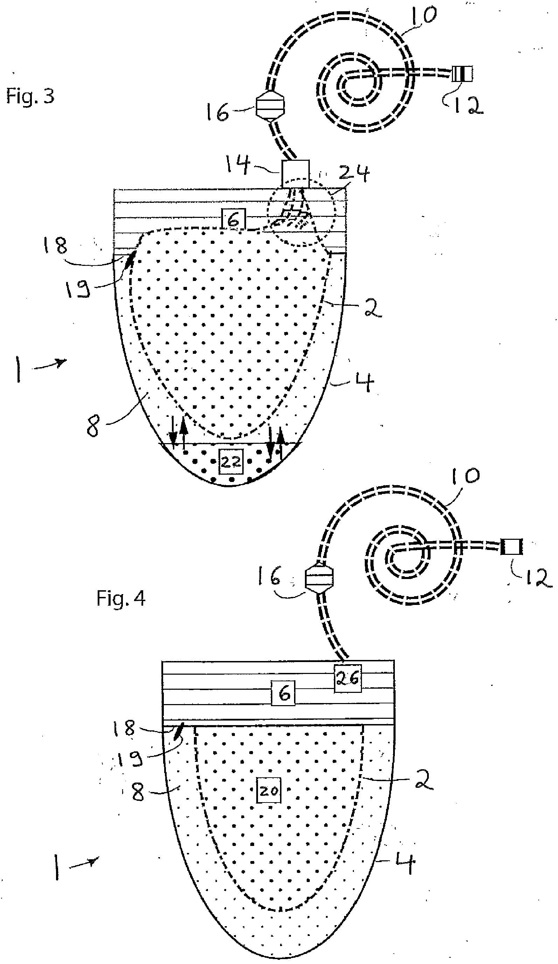

Der Innenbehälter 2 steht im Ausführungsbeispiel über einen Infusionsschlauch 10 mit einem Anschluss 12 in Verbindung, der z.B. als Teil einer Luer-Lock-Verbindung gestaltet ist und im Ausführungsbeispiel auch eine Absperreinrichtung aufweist. Der Infusionsschlauch 10 geht von einer Durchfluss-Einstelleinrichtung 14 aus, in die der Innenbehälter 2 einmündet (siehe Figur 3; das Flüssiggasreservoir 6 hat in seinem inneren unteren Bereich eine Aussparung für den oberen Bereich des Innenbehälters 2). Ferner ist in den Infusionsschlauch 10 ein Partikelfilter 16 eingesetzt.

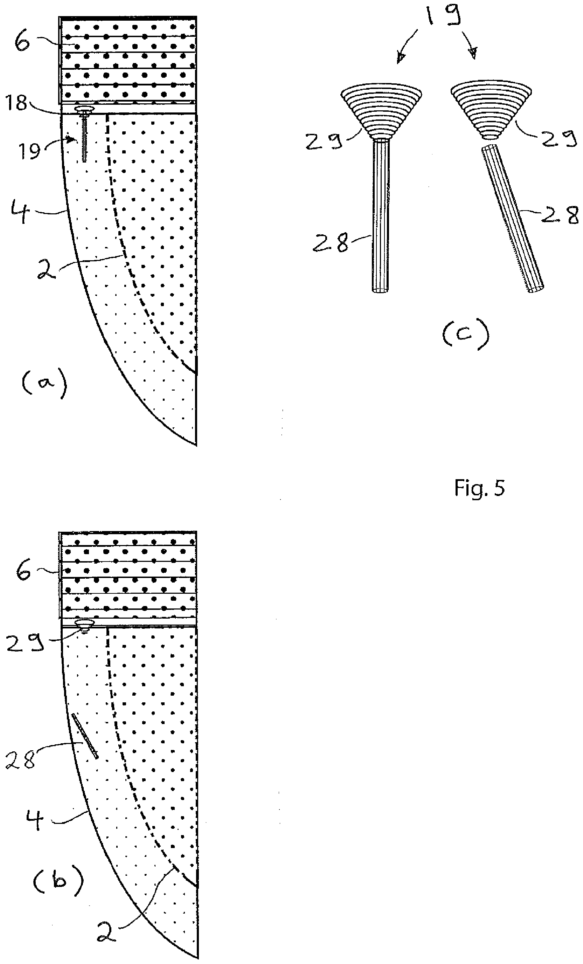

Das Flüssiggasreservoir 6 weist einen in den Zwischenraum 8 führenden Auslass 18 auf. Im Auslieferungszustand der Fluidliefervorrichtung 1 ist dieser Auslass 18 geschlossen (siehe Figur 1). Mit Hilfe einer von außen betätigbaren Öffnungseinrichtung 19 lässt sich der Auslass 18 öffnen. Im Ausführungsbeispiel ist die Öffnungseinrichtung 19 als ein Kunststoffteil mit einer Sollbruchstelle ausgestaltet, das unter einer tangential wirkenden Kraft bricht und den Auslass 18 irreversibel freigibt. Anhand von Figur 5 sind weiter unten Ausführungsformen für die Öffnungseinrichtung 19 näher erläutert.

Im Folgenden wird anhand der Figuren 1 bis 3 erläutert, wie man im Ausführungsbeispiel die Fluidliefervorrichtung 1 benutzen kann.

Im Auslieferungszustand der Fluidliefervorrichtung 1 befindet sich im Innenbehälter 2 bereits ein Fluid 20 (hier eine Infusionslösung). Ferner ist das allseitig geschlossene Flüssiggasreservoir 6 mit einem Flüssiggas gefüllt. Das Volumen des Flüssiggasreservoirs 6 ist so bemessen, dass der größte Teil des Volumens von Flüssigkeit und nur ein kleinerer Teil von dem Gas eingenommen wird, das im Gleichgewicht mit dem flüssigen Anteil des Flüssiggases steht.

Als Flüssiggas eignen sich z.B. 1.1.1.2.3.3.3-Heptafluorpropan, CF₃CHFCF₃, das bei einem Druck von 1,013 bar einen Siedepunkt von -16,4°C und bei einer Temperatur von 20°C einen Dampfdruck von 3,90 bar hat, oder 1.1.1.2-Tetrafluorethan, CH₂FCF₃, das bei einem Druck von 1,013 bar einen Siedepunkt von -26,1°C und bei einer Temperatur von 20°C einen Dampfdruck von 5,72 bar hat, oder auch andere Hydrofluoralkane oder im Vergleich dazu preisgünstigere Alkane wie Propan, n-Butan oder Isobutan.

Wenn nun bei geschlossenem Anschluss 12 der Auslass 18 des Flüssiggasreservoirs 6 mit Hilfe der Öffnungseinrichtung 19 geöffnet wird, fließt die flüssige Phase 22 des Flüssiggases in den unteren Bereich des Zwischenraums 8, wie in Figur 2 gezeigt. Da der Zwischenraum 8 ein zusätzliches Volumen bereitstellt, verdampft ein Teil des Flüssiggases 22 und füllt den Zwischenraum 8. Die erforderliche Verdampfungswärme wird durch Wärmetransport von innen über das Fluid 20 (das eine vergleichsweise hohe Wärmekapazität hat) und von außen zugeführt. Es kann einige Zeit (z.B. ein paar Minuten) dauern, bis sich ein Gleichgewicht eingestellt hat und sich über der flüssigen Phase des Flüssiggases 22 eine Gasphase ausgebildet hat, die auf den flexiblen Innenbehälter 2 als Druck den Dampfdruck des Flüssiggases 22 ausübt.

Die Fluidliefervorrichtung 1 ist nun gebrauchsfertig, so dass der Anschluss 12 an das Gegenstück der Luer-Lock-Verbindung angeschlossen werden kann, das sich im Ausführungsbeispiel am Ende einer Infusionskanüle bei einem Patienten befindet. Nach Öffnen des Anschlusses 12 strömt das Fluid 20 von dem Innenbehälter 2 zu dem Anschluss 12. Die Durchfluss-Einstelleinrichtung 14 ist dabei so eingerichtet, dass sich ein gewünschter Durchfluss (Förderrate) durch den Infusionsschlauch 10 ergibt. Mit Hilfe der Durchfluss-Einstelleinrichtung 14 wird der Strömungswiderstand in der Verbindung zwischen dem Innenbehälter 2 und dem Anschluss 12 festgelegt. Solange der auf den Innenbehälter 2 wirkende Druck konstant ist, ist auch der Durchfluss durch den Infusionsschlauch 10 konstant. Dieser Druck ist der Dampfdruck des Flüssiggases 22, der sich nicht ändert, wenn der Zwischenraum 8 mit abnehmendem Inhalt des Innenbehälters 2 größer wird.

In Figur 3 ist schematisch gezeigt, wie eine mit 24 bezeichnete Sicherheitseinrichtung verhindert, dass bei entleertem Innenbehälter 2 Flüssiggas in den Infusionsschlauch 10 übertreten kann, wie es grundsätzlich passieren könnte, wenn der Innenbehälter 2 defekt ist, z.B. durch einen von dem Flüssiggas 22 ausgehendem Angriff porös wird. Wenn der Innenbehälter 2 praktisch leer ist (was in Figur 3 noch nicht dargestellt ist), legt sich nämlich seine Wandung in der als Sicherheitseinrichtung 24 dienenden Zone vor der Durchfluss-Einstelleinrichtung 14 gegeneinander, so dass der Auslass aus dem Innenbehälter 2 großflächig blockiert ist und auch kein Flüssiggas 22 in den Infusionsschlauch 10 gelangen kann.

Die Figur 4 zeigt in schematischer Ansicht eine Variante der Ausführungsform aus Figur 1. In Figur 4 sind für einander entsprechende Teile dieselben Bezugszeichen verwendet wie in Figur 1. Die Durchfluss-Einstelleinrichtung 14 aus Figur 1 ist bei der Variante durch eine Durchfluss-Regeleinrichtung 26 ersetzt (die auch eine Absperreinrichtung enthalten kann). Die Durchfluss-Regeleinrichtung 26 befindet sich im oberen Bereich des Flüssiggasreservoirs 6 und steht wegen des geöffneten Auslasses 18 mit dem Zwischenraum 8 in Verbindung. Sie erfasst den Dampfdruck und ist dazu eingerichtet, bei steigendem Druck des Gases im Zwischenraum 8 eine Durchlassöffnung im Bereich der Durchfluss-Regeleinrichtung 26 zu verkleinern und entsprechend bei sinkendem Druck zu vergrößern. Dies kann zum Beispiel mit einem hohlen, balgartigen Zapfen erreicht werden, der in diese Durchlassöffnung hineinragt und in dessen Innenraum der Dampfdruck herrscht. Wird der Dampfdruck größer, verlängert sich der balgartige Zapfen und verkleinert die Durchlassöffnung, und umgekehrt. Derselbe Effekt kann mit einer Durchfluss-Regeleinrichtung erreicht werden, die eine poröse, komprimierbare Struktur aufweist. Die Durchfluss-Regeleinrichtung 26 sorgt dafür, dass sich z.B. temperaturbedingte Schwankungen des Dampfdrucks nicht oder nur in geringem Maße auf die Förderrate des Fluids auswirken.

Der Außenbehälter 4 und das Flüssiggasreservoir 6 müssen ausreichend stabil sein, um dem Dampfdruck des Flüssiggases 22 standzuhalten, wobei auch noch eine Sicherheitsreserve vorliegen muss. Ferner dürfen die Materialien des Außenbehälters 4 und des Flüssiggasreservoirs 6 nicht durch das Flüssiggas 22 angegriffen werden. Geeignete Materialien sind zum Beispiel Polycarbonat (PC) oder Polymethylmethacrylat (PMMA).

Da der Innenbehälter 2 flexibel ist, ist der Druck in seinem Innenraum genauso groß wie außerhalb, so dass der Innenbehälter 2 nicht druckfest sein muss. Das Material des Innenbehälters 2 muss aber mit dem Fluid 20 verträglich sein und gegebenenfalls dafür zugelassen sein. Ferner muss es gegen das Flüssiggas zumindest für eine begrenzte Zeitdauer beständig sein. Vorzugsweise ist das Material des Innenbehälters 2 transparent, so dass man die Restmenge an Fluid 20 in der Fluidliefervorrichtung 1 schnell erfassen kann. Als Material für den Innenbehälter 2 eignet sich zum Beispiel Polyamid (PA).

Bei einer weiteren Ausführungsform der Fluidliefervorrichtung, die in den Figuren nicht dargestellt ist, ist nicht nur der Innenbehälter flexibel, sondern auch der Außenbehälter. Das Flüssiggasreservoir ist starr, kann im Prinzip aber auch flexibel sein. Bei dieser Ausführungsform kann im Auslieferungszustand der Fluidliefervorrichtung der Außenbehälter zumindest weitgehend an dem Innenbehälter anliegen, so dass der Zwischenraum zwischen dem Innenbehälter und dem Außenbehälter klein ist. Diese Ausführungsform eignet sich besonders für Anwendungen, bei denen der Benutzer den Innenbehälter über den Anschluss selbst mit einer vorgegebenen Menge an Fluid befüllt. Diese Menge kann auch gering sein und ist nur durch das maximale Aufnahmevermögen des Innenbehälters begrenzt. Bei dem Befüllvorgang dehnt sich der Innenbehälter aus, und der Außenbehälter macht diese Bewegung mit, denn er verhält sich ähnlich wie ein zusammengelegter Beutel. Wenn nun der Auslass am Flüssiggasreservoir geöffnet wird, wird der flexible Außenbehälter unter dem Dampfdruck des Flüssiggases prall, d.h. der Zwischenraum wächst. Dann übt der Dampfdruck des Flüssiggases den gewünschten Druck auf den Innenbehälter aus, und diese Fluidliefervorrichtung kann analog zu den anhand der Figuren 1 bis 4 erläuterten Ausführungsformen verwendet werden. Der Außenbehälter muss auch in diesem Fall dem Dampfdruck standhalten.

In Figur 5 ist eine Ausführungsform für eine Öffnungseinrichtung 19 genauer dargestellt. Figur 5(a) zeigt den Zustand, in dem der Auslass 18 des Flüssiggasreservoirs 6 geschlossen ist und Figur 5(b) den Zustand nach dem Öffnen. In Figur 5(c) ist die Öffnungseinrichtung 19 vor und kurz nach dem Öffnen vergrößert dargestellt.

Die Öffnungsvorrichtung 19 weist einen Schaft 28 auf, der von einem Kegelteil 29 ausgeht und einstückig mit dem Kegelteil 29 hergestellt ist. Das Kegelteil 29 ist in abgedichteter Weise in eine Öffnung im Bodenbereich des Flüssiggasreservoirs 6 eingesetzt und hat an seinem unteren Ende den Auslass 18 des Flüssiggasreservoirs 6. Der Auslass 18 ist vor dem Öffnen durch den Schaft 28 verschlossen. Wenn der Außenbehälter 4 flexibel ist, lässt sich zum Öffnen des Auslasses 18 der Schaft 28 einfach durch Anwendung eines äußeren Drucks mit der Hand wegbrechen, so dass der Auslass 18 freigegeben wird. Dieser Vorgang wird erleichtert, wenn der Schaft 28 an seinem oberen Ende eine Sollbruchstelle aufweist.

Bei einer festen Form des Außenbehälters 4 eignet sich eine Ausführungsform für die Öffnungseinrichtung, bei der der Schaft länger ist und vorzugsweise bis zum Boden des Außenbehälters reicht, so dass zum Wegbrechen des Schafts (vorzugsweise an einer Sollbruchstelle) eine größere Hebelkraft einwirken kann. Der Schaft kann in diesem Fall mit Hilfe des Gewichts des gefüllten Innenbehälters weggebrochen werden, indem die gefüllte Vorrichtung kontrolliert ruckartig geschwenkt wird. Bei einer Variante dieser Ausführungsform ist der Schaft werksseitig mit dem Innenbehälter verbunden. In dem Maße, wie der Innenbehälter befüllt wird, übt er durch Volumenzunahme eine zunehmende Spannung auf den Schaft aus, was den anschließenden Öffnungsvorgang erleichtert.

## Patentansprüche

1. Fluidliefervorrichtung, insbesondere für Medikamente in flüssiger Form,
- mit einem flexiblen Innenbehälter (2), der zur Aufnahme eines Fluids (20) eingerichtet ist und mit einem Anschluss (12) in Verbindung steht,
- mit einem Außenbehälter (4), der den Innenbehälter (2) zumindest teilweise umgibt, wobei zwischen dem Außenbehälter (4) und dem Innenbehälter (2) ein gasdichter Zwischenraum (8) angeordnet ist und der Anschluss (12) von außen her zugänglich ist,
- mit einem Flüssiggasreservoir (6), das einen in den Zwischenraum (8) führenden Auslass (18) aufweist, und
- mit einer von außen betätigbaren Öffnungseinrichtung (19) für den Auslass (18).

2. Fluidliefervorrichtung nach Anspruch 1, **dadurch gekennzeichnet, dass** das Flüssiggasreservoir (6) mit Flüssiggas (22) befüllt ist.

3. Fluidliefervorrichtung nach Anspruch 2, **dadurch gekennzeichnet, dass** das Flüssiggas (22) mindestens eine der aus der folgenden Liste ausgewählten Substanzen aufweist: Hydrofluoralkane, Propan, n-Butan, Isobutan.

4. Fluidliefervorrichtung nach einem der Ansprüche 1 bis 3, **gekennzeichnet durch** eine Durchfluss-Einstelleinrichtung (14), die zum Einstellen des Durchflusses des von der Fluidliefervorrichtung (1) über den Anschluss (12) gelieferten Fluids (20) eingerichtet ist.

5. Fluidliefervorrichtung nach einem der Ansprüche 1 bis 4, **gekennzeichnet durch** eine Durchfluss-Regeleinrichtung (26), die mit dem Zwischenraum (8) in Verbindung steht und dazu eingerichtet ist, bei steigendem Druck des Gases im Zwischenraum (8) eine Durchlassöffnung in der Verbindung zu dem Anschluss (12) zu verkleinern.

6. Fluidliefervorrichtung nach einem der Ansprüche 1 bis 5, **gekennzeichnet durch** eine Sicherheitseinrichtung (24), die dazu eingerichtet ist, einen Übertritt von Flüssiggas (22) zu dem Anschluss (12) zu verhindern.

7. Fluidliefervorrichtung nach einem der Ansprüche 1 bis 6, **dadurch gekennzeichnet, dass** der Innenbehälter (2) mit einem Fluid (20) befüllt ist, das mindestens einen der folgenden Bestandteile enthält: eine Flüssigkeit, ein Pulver, eine Emulsion, eine Suspension, ein Gas, eine thixotrope Flüssigkeit.

8. Fluidliefervorrichtung nach Anspruch 7, **dadurch gekennzeichnet, dass** der Innenbehälter (2) mit einem Fluid (20) befüllt ist, das eine pharmazeutische Zusammensetzung aufweist.

9. Fluidliefervorrichtung nach Anspruch 8, **dadurch gekennzeichnet, dass** das Fluid (20) mindestens eine der aus der folgenden Liste ausgewählten Substanzen enthält: Analgetika, Antibiotika, Antipsychotika, Blutkomponenten, Chemotherapeutika zur Behandlung von Krebserkrankungen, monoklonale Antikörper, Sedativa, Supportiva für die parenterale Ernährung, Supportiva für die enterale Ernährung, pharmazeutische Zubereitungen zur Behandlung von Stoffwechselerkrankungen oder Autoimmunerkrankungen, pharmazeutische Zubereitungen für die Durchführung von Zelltherapien.

10. Fluidliefervorrichtung nach einem der Ansprüche 1 bis 9, **dadurch gekennzeichnet, dass** der Außenbehälter (4) eine feste Form hat.

11. Fluidliefervorrichtung nach Anspruch 10, **dadurch gekennzeichnet, dass** der Zwischenraum (8) im Auslieferungszustand der Fluidliefervorrichtung (1) mit Gas entsprechend dem Flüssiggas (22) zur Bereitstellung eines Gegendrucks gegen den äußeren Luftdruck gefüllt ist.

12. Fluidliefervorrichtung nach einem der Ansprüche 1 bis 9, **dadurch gekennzeichnet, dass** der Außenbehälter flexibel ist.

13. Fluidliefervorrichtung nach Anspruch 12, **dadurch gekennzeichnet, dass** der Außenbehälter vor Betätigung der Öffnungseinrichtung (19) für den Auslass (18) zumindest teilweise an dem Innenbehälter (2) anliegt.

14. Fluidliefervorrichtung nach einem der Ansprüche 1 bis 13, **dadurch gekennzeichnet, dass** der Innenbehälter (2) und/oder der Außenbehälter (4) transparentes Kunststoffmaterial aufweisen.

15. Fluidliefervorrichtung nach einem der Ansprüche 1 bis 14, **dadurch gekennzeichnet, dass** der flexible Innenbehälter (2) über eine Infusionsleitung (10) mit dem Anschluss (12) in Verbindung steht, wobei die Infusionsleitung (10) vorzugsweise mit einem Durchflussbegrenzer und/oder einem Partikelfilter (16) versehen ist und wobei der Anschluss (12) vorzugsweise als Teil einer Luer-Lock-Verbindung gestaltet ist.
